# EUROPÄISCHE PATENTANMELDUNG

(11) **EP 4 056 530 A1**
(43) Veröffentlichungstag der Anmeldung: **14.09.2022**
(21) Anmeldenummer: 22161189.0
(22) Anmeldetag: 09.03.2022
(51) Int. Cl.: C01B 3/22, C01B 3/24, C01B 3/04, C12P 5/00

(54) **VERFAHREN ZUR BEHANDLUNG VON FLÜSSIGKEITEN UND / ODER SUSPENSIONEN UND ZUR ERZEUGUNG VON WASSERSTOFF AUS WASSERSOFF-ENTHALTENDEN CHEMISCHEN VERBINDUNGEN**

(30) Priorität: 09.03.2021 DE 102021105704
(71) Anmelder: PRE Power Recycling Energyservice GmbH, 17033 Neubrandenburg Mecklenburg-Vorpommern (DE)
(72) Erfinder: ROSSOW, Norbert, 17217 Penzlin (DE)
(74) Vertreter: Wehlan, Martin

(57) **Zusammenfassung**

Die Erfindung betrifft ein Verfahren und eine Vorrichtung zur Behandlung von Flüssigkeiten und / oder Suspensionen und zur Erzeugung von Wasserstoff aus wassersoff-enthaltenden chemischen Verbindungen, gekennzeichnet durch folgende Schritte:
▪ Vorbehandlung der Flüssigkeiten und / oder Suspensionen mittels Ultraschall
▪ Behandlung der Flüssigkeiten und / oder Suspensionen mittels einer Kombination aus Ultraschall und Plasma
▪ Nachbehandlung der Flüssigkeiten und / oder Suspensionen mittels eines Plasmas.

Die Flüssigkeiten und / oder Suspensionen können aus Biogasanlagen oder anderen Fermentationsanlegen entnommen werden und organische und / oder stickstoffhaltige Bestandteile enthalten.

## Beschreibung

Die Erfindung betrifft eine Vorrichtung und ein Verfahren zur mehrstufigen bzw. zyklischen Behandlung von organische Bestandteile enthaltenden Flüssigkeiten zur Extraktion von Wasserstoff und dessen Verwendung in einem geschlossenen Verfahrensablauf auf der Basis von Ultraschall und Plasma erzeugenden Komponenten, das eine Verbesserung gegenüber der Erfindung darstellt, welche in der bis zum 9. März 2021 unveröffentlichten europäischen Patentanmeldung Az. 20194897.3 beschrieben wurde. Der Kern der Erfindung besteht darin, durch die Kombination von Plasma und Ultraschall thermische und physikalische sowie chemische Bedingungen bereitzustellen, bei denen Prozesse zur Dampfreformierung und der thermischen Dissoziation (Abspaltung von Wasserstoff aus wasserstoffhaltigen chemischen Verbindungen) erzeugt werden.

### Stand der Technik

Weit verbreitet in der Anwendung des Ultraschalls in flüssigen bzw. fließfähigen Medien und Stoffen sind Ultraschallgeräte bzw. Systeme, die auf indirekter Beschallung durch auf Trägerflächen aufgebrachte Schwinger bzw. Schwinggebilde, welche einen indirekten Eintrag in die Behandlungsstoffe und deren Ströme im fließenden bzw. deren Behälter im Batchverfahren ausüben.

Verfahren bzw. Geräte, die mit direktem Kontakt zu den zu behandelnden flüssigen bzw. fließfähigen Medien arbeiten, haben zu den Schwingkörpern des Ultraschall-Bauteils ein Anströmungs- bzw. Umströmungs-System aus Kammern oder Rohrleitungen, welche die zyklische oder kontinuierliche Beschallung ermöglichen.

Bei diesen derzeitig etablierten Verfahren und Geräten der indirekten Beschallung ist die Effizienz der Behandlung für verschiedene Verfahren der z.B. Oberflächen-Behandlung bzw. Reinigung bei technischen Anwendungen in der Medizin und der Behandlung von kleinteiligen Instrumenten etc. ausreichend effizient.

Bei Verfahren mit großen zu behandelnden Volumenströmen und hohen Anforderungen bzgl. der stofflichen Desintegration haben diese Verfahren klare Nachteile gegen über den direkt in der Suspension arbeitenden Verfahren und Geräten.

Geräte der Ultraschallbehandlung mit direktem Schalleintrag in die Suspension haben eine Position erreicht, welche sie anderen Verfahren zur Desintegration in der Abwasserbehandlung und der Gärrestbehandlung deutlich energieeffizienter und wirkungsvoller nutzbar machen.

Bei großen zu behandelnden Volumenströmen wie in Abwasserbehandlungs- und anaeroben Vergärungsanlagen gibt es derzeitig große Defizite bzgl. der Aufspaltung von Schadstoffen und organischen Inhaltsstoffen. Die Effizienz der vorhandenen Technik und Systeme reicht nicht für die zukünftigen Aufgaben der Behandlung von Schadstoffen und Giften im humanen wie industriellen Abwasser auf wirtschaftlicher Basis.

Zum Stand der Technik gehören auch die folgenden deutschen Patent- bzw. Offenlegungsschriften:

Die DE 102 30 897 A1 beschreibt thermohydraulische Schallquellen, die Kugelschalenförmige Elektroden besitzen, zwischen welchen die Kavitation erzeugt werden soll. Piezoelektrische angetriebenen Ultraschallschwinger werden dort nicht genannt. In DE 20 2012 010 605 U1 wird eine hochfrequente mechanische Schwingungsquelle offenbart, um Gase aufzuspalten und abzuführen. Sie dient der Gewinnung von Wasserstoff und Sauerstoff. Es wird keine piezoelektrisch erzeugte Schwingung über Booster und Horn erwähnt und keine direkt in einer strömenden Flüssigkeit wirkende Gestaltung.

Bei DE 10 2016 002 599 A1 handelt es sich um ein Verfahren zum Separieren von Feststoff Partikeln aus einer Flüssigkeit. Dies soll durch das Einbringen von nicht spezifizierten Schallwellen erfolgen. Die Schallwellen werden siehe Anspruch 2, quer zur Förderrichtung eingebracht, nicht gegen die Strömungsrichtung. In dieser Schrift geht es nicht um Aufschlüsse von Stoffen, sondern um das Separieren von Feststoffpartikeln.

WO 2014/181284 A1 beschreibt die Vergrößerung der Menge von Polyphenolen im Olivenöl bei dessen Herstellung mittels Ultraschalles. Dabei sind die Sonotroden nicht in der Flüssigkeit, sondern auf die "external Surface" auf der Außenseite angebracht.

DE 600 01 230 T2 (EP 1173387) offenbart ein Verfahren, das zum Desinfizieren und Reinigen von Flüssigkeiten und Gasen dient.

In EP 1 276556 B1 wird die Ultraschallbehandlung durch Ultraschall-produzierenden Transducer bzw. Wandler durchgeführt, die asymmetrisch um ein Liniensegment angeordnet sind. Der Frequenzbereich beträgt hauptsächlich 0,5 und 5 MHz, nicht um 20 kHz.

Teilweise zum Stand der Technik gehört auch die zum Prioritätszeitpunkt unveröffentlichte Druckschrift EP 3 603 812 A1. Dort werden Suspensionen mittels Ultraschall und Plasma behandelt. Eine besonders gestalteter Reaktionsraum wird dort nicht beschrieben.

Zur Erfüllung des Pariser Klimaschutzabkommens hat sich die Bundesregierung verpflichtet die Treibhausgasemissionen in verschiedenen Sektoren der Wirtschaft und der Landwirtschaft um 38% gegenüber 2005 zu vermindern.

Arbeitsgrundlage für dieses Ziel ist das Klimaschutzprogramm 2030.

Auf diesem Weg steht die Behandlung von organischen Flüssigkeiten und Suspensionen aus verschiedenen Abfallquellen eine große Rolle.

Diese organischen Abfälle bedürfen einer umfangreichen Behandlung, um eine Umwandlung der enthaltenen Schadstoffe zu nutzbaren Stoffen bzw. deren Nutzung zur Vermeidung schädlicher Einflüsse zu erreichen.

Organisch belastete Abwässer oder Wirtschaftsdünger der Landwirtschaft bilden zum einen eine enorme Schadstofffracht, bilden aber auf der anderen Seite ein gewaltiges Potential zur stofflichen und energetischen Nutzung.

Um die Nutzung zu erlangen und wirtschaftliche Potentiale zu erschließen, werden weltweit in Abwasserbehandlungsanlagen und Biogasanlagen der Abfall- wie der Landwirtschaft anaerobe Behandlungsverfahren praktiziert.

Um die Effizienz der Verfahren zur Behandlung und Nutzung der anaerob aufschließbaren Potentiale zu erhöhen werden seit mehreren Jahren Geräte zur Behandlung und zum Aufschluss der organischen Bestandteile der Abwasser- bzw. Abfallströme mit Ultraschall behandelt.

Diese Verfahren und Geräte beruhen auf dem physikalisch bewirkten Aufschluss der organischen Inhaltsstoffe in eine Partikel bzw. Molekülform, welche den biologischen Abbauprozess mit unterschiedlichen Zielstellungen verstärkt.

Plasmachemische Verfahren werden in der Behandlung von flüssigen Strömen der o.g. Umwelttechnikbereiche bisher nur in indirekter Einwirkung bzw. als Ozoneinleitung benützt.

In dem hier beschriebenen Verfahren und der die Basis bildenden Vorrichtung geht es um die verfahrenstechnische Verknüpfung und stufenweise Koppelung von den in der Flüssigkeit erzeugten Plasmen mit der direkt in den Flüssigkeiten vorgenommenen Schwingungseintragung von Ultraschallquellen.

### Aufgabe der Erfindung

Der Nachteil der bisher bekannten technischen Lösung besteht darin, dass es bisher die Beeinflussung und Steuerung des Primärprozesses der Methanproduktion und der zusätzlichen CO2 Umwandlung mittels Wasserstoff in Methan sowie der damit verbundenen Entgiftung des Primärprozesses bzgl. Ammoniak und Schwefelwasserstoff(siehe Anlage zur Mail zuvor) nur durch externe Prozesse und aus externen Quellen gibt.

### Lösung der Aufgabe

Die Aufgabe wurde gemäß den Merkmalen der Patentansprüche gelöst.

### 1. Allgemeine Verfahrensbeschreibung

### 1.1 Beschreibung

Diese Reaktionsräume werden von organischen flüssigen Medien durchströmt.

Diese Reaktionsräume werden für unterschiedliche Stufen des Prozesses mit Reaktionszonen der solo Ultraschall und solo Plasmaerzeugung sowie von gemeinsam von Plasmen und Ultraschall gestalteten Reaktionszonen durchströmt.

Bei der Behandlung von organische Bestandteile enthaltenden Flüssigkeiten kann durch Ultraschallbehandlung eine Aufspaltung von organischen Konglomeraten, Fasern und Partikeln bis in die Molekularebene erfolgen.

Basis hierfür ist, die durch Ultraschall ausgelöste Kavitation mit ihren physikalisch, chemisch und biologisch Wirkungen.

Werden diese Wirkungen mit durch Plasmen in den Flüssigkeiten der Ultraschallbehandlung ausgelösten Vorgängen der Plasma-Chemie und Physik verknüpft, so ergeben sich die beabsichtigten Wirkungen der Ultraschall wie der Plasma Behandlung.

Um das Verfahren in seinen Schritten näher zu erläutern wird die zu behandelnde Flüssigkeit flüssiger Biomasse betrachtet.

Ein erster Verfahrensschritt der Behandlung flüssiger Biomasse dient dem weiteren Aufschluss der in der Flüssigkeit bestehenden Strukturen der organischen Bestandteile.

Hierzu wird maßgeblich Ultraschall aus von der Flüssigkeit überströmten Ultraschall abstrahlenden Bauteilen gefördert. Dadurch entsteht ein direkter Aufschluss im Sinne der Ultraschall relevanten Wirkungen.

Mit dieser ersten Verfahrensstufe wird die Voraussetzung für eine sehr effektive kombinierte Behandlung der Biomasse geschaffen.

Der hohe Grad des Aufschlusses aus der ersten Stufe bildet die Voraussetzung für eine hohe Wirksamkeit der Einwirkung des Ultraschalls gemeinsam mit dem Plasma in einem sich überschneidenden und ergänzenden Behandlungsraum, in dem sich sowohl die Ultraschall Schwingungen als auch die Plasma Bereiche ausbreiten, überschneiden und ergänzen.

Durch diese Einwirkung beider Technologien auf das zu behandelnde Substrat der Biomasse verstärken sich Effekte zum Aufschluss und zur Auslösung der speziellen physikalischen, Biochemischen sowie rein chemischen Wirkungen in überproportionaler Stärke gegenüber den Wirkungen aus der einzeln eingesetzten Technologie des Ultraschalls bzw. des Plasmas.

Dieser Bereich der Behandlung stellt die zweite Stufe dar.

Diese Verfahrensstufen können variabel einzeln, wiederholt oder in unterschiedlicher Folge durchgeführt werden.

### 1.2 Behandlungssubstrat Zuführung

Das zu behandelnde Substrat bzw. Flüssigkeit wird aus aktiven Reaktionsbehältern bzw. Reaktionsgefäßen in denen die zu behandelnden Substrate gezielten anderen Reaktionen wie zum Beispiel der Fermentation ausgesetzt sind mittels Zuführeinrichtungen wie z.B. Pumpen entnommen.

Alternativ kann das Substrat auch aus Lagerbehältern ohne eine aktive Reaktionsbestimmung auf ähnliche Weise kontinuierlich oder zyklisch entnommen werden.

Für aktive Behälter steht beispielhaft die Entnahme aus einem Biogas produzierenden Behältersystem. Für inaktive Lagerbehälter steht zum Beispiel ein Behälter für Prozessabwässer oder Wirtschaftsdüngerlagerung.

### 1.3 Behandlungssubstrat Abführung

Nach der Behandlung des Substrates entsprechend 1.1 wird das Substrat dem Ausgangsbehälter bzw. System erneut zugeführt (Bypass Behandlung), um die Hauptreaktion weiterzuführen bzw. einer weiterführenden Prozessstufe zugeführt (Durchfluss Behandlung).

Diese Abführung des Substrates nach erfolgter Behandlung erfolgt mittels Pumpen, Injektionsstrahlsystemen oder durch abfließen mittels geodätischen Höhenunterschiedes.

### 1.4 Systemaufbau

### 1.4.1 Wirkbereiche für Ultraschallbehandlung

Sind vom zu behandelnden Medium durchströmte Rohre bzw. Einrichtungen unterschiedlicher Form, Dimension und Geometrie welche einen geschlossenen Abschnitt des Verfahrens darstellen.

Diese Abschnitte sind dadurch gekennzeichnet, dass sie gegen die Umgebende Atmosphäre und gegen umgebende Flüssigkeiten abgeschlossen sind.

In dem durchströmten Raum dieser Bereiche befinden sich Emitter verschiedener Ultraschallquellen mit variierenden oder gleichen Eigenschaften bzgl. ihrer Leistung, Frequenz und Amplitude.

Das zu behandelnde Medium umströmt die emittierenden Ultraschallquellen direkt, indirekt, kontinuierlich oder zyklisch.

### 1.4.2 Wirkbereiche für Plasmabehandlung

Die Wirkbereiche der Plasmabehandlung entsprechen in ihrer Beschreibung denen der Ultraschall-Behandlung mit dem Unterschied, dass es sich hier um die Anordnung von Plasmaquellen gleichen oder unterschiedlichen technischen Ursprungs handelt.

So können hier je nach Art, Qualität und Eigenschaften des zu behandelnden Mediums kalte und thermische Plasmen unterschiedlichen Ursprungs eingesetzt werden.

Hier handelt es sich beispielhaft um Spark-Plasmen, Corona Plasmen, Microwellen Plasmen oder Torch-Plasmen, welche sich direkt in der zu behandelnden Flüssigkeit ausbilden.

### 1.4.3 Wirkbereiche für kombinierte US - Plasmabehandlung

Die Wirkbereiche der kombinierten Behandlung entsprechen in ihrer Beschreibung aus 1.4.1 und 1.4.2 mit dem Unterschied, dass die Ultraschall Emitter und die Plasmen ausbildenden Bauteile so angeordnet sind, dass ihre Hauptreaktionsbereiche, der Ausbildung der Hauptkavitationszonen und die Bereiche in denen sich das Plasma räumlich ausbildet angrenzend oder überlagernd sind.

### 2. Vorrichtung zur mehrstufigen Behandlung organischer Suspensionen und Flüssigkeiten zur Wasserstoff Extraktion und damit verbundenen Behandlung des Substrates

### 2.1. Aufbau des Gerätes

Das Gerät besteht aus folgenden Hauptbaugruppen:

### 2.1.1 Substratführungs- und Behandlungsbereich

Rohrleitungsteile und Reaktionsbereiche für die Durchströmung und Behandlung des zu behandelnden Substrates

In diesen Bauteilen erfolgt die Zu- und Abfuhr bzgl. der Behandlungs- und Reaktionsbereiche für das Substrat, die Aufnahme und Abfuhr von durch die Behandlung auftretende Sedimente sowie die gezielte Abführung der entstehenden ungelösten Gase bzw. Gasanteile.

### 2.1.2 Armarturen, Messgeräte und Feldgeräte für Prozess und Sicherheitsfunktionen

### 2.1.3 Bauteile zur Temperierung, Belüftung und Klimatisierung der substratführenden

### Anlagenteile sowie des Raumes in der Gerätehülle

### 2.1.4. Gerätehülle

Einhausung der Anlagenbauteile zur Abschirmung gegenüber äußeren Einflüssen sowie der Vermeidung negativer Einflüsse auf die Umgebung des Aufstellortes.

### 2.1.5. Automation und Steuerung

Bauteil zur Aufnahme aller für die Steuerung und Regelung des Prozesses der verknüpften Elemente des Gerätes erforderlichen Elektrotechnischen Teile.

Es beinhaltet Generatoren, SPS Geräte und Software.

Die Prozesssteuerung, Datenfernübertragung Datenarchivierung und Gewährleistung der Anlagensicherheit sind die integrierten Aufgaben.

### 2.2 Funktion des Gerätes

Das Gerät ist durch Pumpen oder andere Fördereinrichtungen für Suspensionen und Flüssigkeiten mit den Behältern oder Anlagen, welche diese bereitstellen verbunden.

Durch die Fördereinrichtungen wird das Substrat den Behandlungsbereichen des Gerätes zugeführt und auch weiterbefördert, aus dem Gerät abgeführt.

Die Durchströmung des Gerätes erfolgt kontinuierlich oder zyklisch

Die in die Suspension eingebrachte Ultraschallenergie und die parallel oder nachträglich zugeführte Plasma basierte Energiezufuhr wird über die Steuerung auf den Volumenstrom oder die zu behandelnde Substratmenge optimiert geregelt.

Die System Temperatur, die Substrattemperatur die Druckverhältnisse und die Gasabfuhr und Behandlung werden durch die Automation geregelt, überwacht und archiviert sowie fernübertragen.

Die Substratbehandlung erfolgt in den drei verschiedenen Reaktionsräume ausbildenden Bereiche welche von der Suspension durchströmt werden.

Die Ultraschallbehandlung erfolgt mittels in die Strömung eingebrachte Erreger zur Kavitationsausbreitung und der damit verbundenen sonophysikalischen zumeist durch Scherkräfte der Implosion gekennzeichnete Aufschluss Effekte bezüglich Partikeln und Molekülen.

Hiermit werden die Oberflächen der in der Suspension erhaltenen partikulären Bestandteile erheblich vergrößert und zu verstärkter Reaktionsfähigkeit aktiviert.

Die Plasmabehandlung erfolgt mittels der Ausbildung von kalten oder thermischen Plasmen in der zu behandelnden Suspension bzw. Flüssigkeit.

Das Plasma bildet partiell Temperaturen von 2000-4000 K aus und bildet damit Reaktionszonen in denen die in dem zu behandelnden Medium enthaltenen Stoffe besondere Reaktionen zeigen.

Durch die partiell auftretenden hohen Temperaturen werden Bedingungen für die Thermische Dissoziation von Molekülen geschaffen.

Organische Bestandteile der zu behandelnden Medien und vor allem Kohlenwasserstoffe und der sich bildende Wasserdampf werden so gespalten, dass sich Wasserstoff direkt abspaltet.

Die entstehenden Bedingungen in und um das sich ausbildende Plasma entsprechen auch den Bedingungen für das Dampfreformierungsverfahren zur Erzeugung von Wasserstoff aus Biomasse.

Die Kombination der Ausbildung von Ultraschall Wirkbereichen und der Ausbildung von Plasmen in dem zu behandelnden Medium zu parallelen und sich partiell überlagernden Wirkbereichen wird in durch das Medium durchströmten Bereichen der aus Rohrleitungen und Reaktionsräumen gebildeten Teilen der Vorrichtung erzeugt.

Durch die Gestaltung solcher Kombinationsbereiche wird die Reaktionszone für die Ausbildung der Effekte beider Energieeintragssysteme multipliziert und eine überproportionale Wirkung der sonophysikalischen und der plasmachemischen Prozesse erzielt.

Dies bedeutet überproportionale Aufschluss Leistung und überproportionale Wirkung der Gasbildungs-, vor allem Wasserstoffbildung aus dem zu behandelnden organischen Medium.

Der Prozess wird durch die Gestaltung der Vorrichtung stufenweise über die Ultraschallbehandlung als Vorbehandlung, die Kombinationsbehandlung als Hauptstufe und die Plasmabehandlung als Nachbehandlungsstufe geführt.

Für die Anpassung an die Besonderheiten unterschiedlicher Suspensionen oder Flüssigkeiten, können die Prozessstufen wiederholt oder in unterschiedlicher Reihenfolge den Suspensionseigenschaften hinsichtlich Aufschlusses organischer Inhaltsstoffe und dem Wasserstoffpotential durchgeführt werden.

Hierzu dient die wiederholte Anordnung von Wirkbereichen mit Reaktionsräumen und die Gestaltung von Bypass Strömen des zu behandelnden Mediums.

Der springende Punkt der Erfindung ist die stufenweise Anordnung von Reaktionsräumen des Ultraschalls zum Zwecke der physikalischen, maßgeblich durch Kavitation herbeigeführten Reaktionen des partikulären Aufschlusses in der ersten Stufe, dann in der zweiten Stufe erfolgt dann die Anordnung der kombinierten Reaktionsräume, des Ultraschall plus Plasma zur Auslösung der im Vergleich zu den mit nur Ultraschall oder nur Plasma ausgestatteten überproportionalen Effekten. Die Effekte sind hier eine überproportionale partikuläre Aufspaltung der organischen Komponenten der Suspension bzw. Flüssigkeit plus, sowie eine überproportionale plasmachemische Reaktion zur molekularen Aufspaltung von organischen Inhaltsstoffen, wie Kohlenwasserstoffe, mittels thermischer Dissoziation und Dampfreformierung sowie der Aufspaltung von Ammonium und Ammoniak sowie Schwefelwasserstoff in die Basiselemente und Wasserstoff.

Die Effizienz des Systems wird durch die Angrenzung und Überlappung der Hauptreaktionszonen des US und des Plasma und deren Abgleich, Regelung der Leistungsparameter beider Systeme mit dem Ziel der Suspension/Flüssigkeits- spezifischen Wasserstoff Abspaltung aus Kohlenwasserstoffen und anderen Wasserstoffhaltigen Verbindungen und letztlich aus in der Suspension bzw. Flüssigkeit enthaltenem Wasser erzielt.

Die zu behandelnde Flüssigkeit durchströmt hierzu gegebenenfalls Reaktionsräume der Stufe eins und der Stufe zwei wiederholt in dieser oder geänderter Reihenfolge, wobei die Parameter des Ultraschall in Frequenz, Leistung und Amplitude variieren und die Plasmen welche sich in der Suspension ausbreiten, aus wechselnden Plasmaquellen wie beispielsweise Microwellenplasmen, Spark-Plasmen oder Torch-Plasmen erzeugt werden.

Das Besondere ist die gezielte Aneinanderreihung von speziellen Reaktionsräumen zur Optimierung der Zielreaktionen zur Aufspaltung der Partikel und Moleküle organischer flüssiger Medien mit dem Ziel der Steigerung der biologischen Effizienz des Primärprozesses, wie der Biogasproduktion in einem Anaerob Reaktor. Diese positive Beeinflussung des Primärprozesses wird maßgeblich durch Steigerung des gelösten CSB Gehaltes sowie der Abspaltung und Gewinnung von Wasserstoff aus primärprozesseigenen Inhaltsstoffen und Verbindungen erzeugt.

In der bisher unveröffentlichten europäischen Patentanmeldung Az. 20194897.3 wurde die prinzipielle Kombination von Ultraschall und Plasma bereits im Labormaßstab demonstriert. In der Praxis zeigt sich aber, dass für Großanlagen weitere Verbesserungen notwendig sind. Es ist aber möglich, auf die in EP-Az. 20194897.3 beschriebenen Konstruktionen zurückzugreifen und diese Anordnungen zu ändern, damit das erfindungsgemäße Ziel - die Erzeugung von Wasserstoff aus wasserstoff-enthaltenden chemischen Verbindungen - mittels Ultraschall und anschließender Kombination von US und Plasma sowie einer Plasmanachbehandlung zu realisieren.

Die Vorrichtung verfügt über geeignete Kommunikationselemente bzgl. der MethanProduktion des Hauptprozesses. Über Software Tools wird die Wasserstoffproduktion des Kombiverfahrens mittels Leistungsregelung dem Aufnahmevermögen der Biomasse Aufbereitungsanlage angepasst.

Das Verfahren ist so skalierbar, dass die Produktion von Wasserstoff aus dem Behandlungs- Medium so stark durch den Aufschluss von Ammoniak in Wasserstoff und prozessunschädlichem Stickstoff erfolgt, dass organische Abfälle oder Wirtschaftsdünger mit toxischem Ammoniak Gehalt in Monofermentationsverfahren durch die Minderung des beschriebenen Verfahrens vergoren werden können bzw. nur eine vergleichsweise geringe Verdünnung der Stoffe in den angestrebten Anaerob anlagen erforderlich wird.

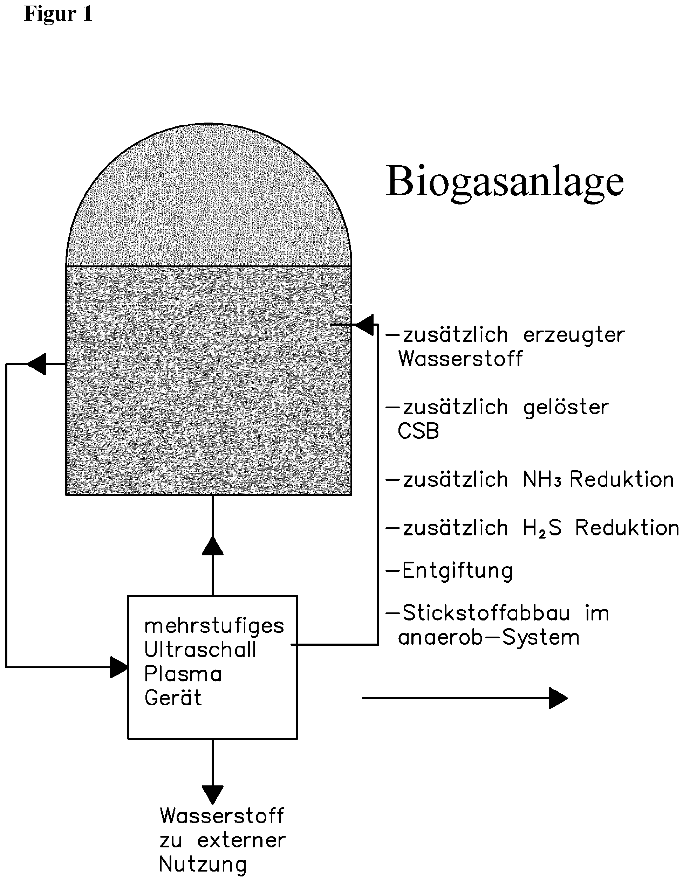

Die Figur 1 zeigt schematisch das erfindungsgemäße Verfahren. Einer Biogas-Anlage wird Flüssigkeit, die organische Bestandteile enthält entnommen. Diese Flüssigkeit wird erst mit Ultraschall, dann kombiniert mit US und Plasma behandelt und letztlich mit Plasma nachbehandelt und wieder der Biogas-Anlage zugeführt. Der erzeugt Wasserstoff kann ebenfalls zur Steigerung der CH₄-Produktion der Biogas-Anlage zugeführt oder aber extern verwendet werden. Außerdem wird durch NH₃-Abbau eine Entgiftung erreicht.

Die erfindungsgemäße Kombination aus US und Plasma hat folgende Vorteile:

### Ultraschall

▪ Desintegration von festen Partikeln und großen Molekülen
▪ Basis Kavitation 5.000·°C; 500bar
▪ Anstieg der gelösten Kohlenwasserstoff-Verbindungen
▪ Anregung der Ionisierung der in Verbindung enthaltenen Stoffe
▪ Beschleunigung der Hydrolyse

### Plasma (beispielsweise Mikrowellenplasma)

▪ partielle Temperaturen von 2.000·K - 4.000·K
▪ Leistungseintrag vorzugsweise 25% von Ultraschall
▪ Überproportionale gelöste CSB Anstiege

### Effekte

▪ Steigerung der Rohstoffausnutzung
▪ Hydrolyse gelöste CSB Steigerung
▪ zusätzliche Methanproduktion durch H₂
▪ potenzielle Gewinnung von grünem H₂ für eine externe Nutzung
▪ Entgiftung durch Abbau von NH₃ und H₂S

### ökologische Effekte

▪ • Nachvergasungspotential starke Minderung
▪ • Ammoniak Emissionen starke Minderung
▪ zusätzlicher Abbau von organischen Kohlenwasserstoffen
▪ Minderung von CO₂ - Emissionen
▪ chemische Speicherung von Strom aus Erneuerbaren Energien
▪ (grüner Strom - grünes Methan)

### Reaktionen der Kombination

▪ überproportionale Steigerung der Hydrolysewirkung gemäß gelöstem CSB Anstieg
▪ starker Desintegrations-Anstieg
▪ Druckaufbau durch Gasbildung
▪ Kohlenwasserstoff C atomar + H₂
▪ Biomasse ähnlich Dampfreforming H₂
▪ Ammonium/Ammoniak N₂ + H₂

## Patentansprüche

1. Verfahren zur Behandlung von Flüssigkeiten und / oder Suspensionen und zur Erzeugung von Wasserstoff aus wassersoff-enthaltenden chemischen Verbindungen, **gekennzeichnet durch** folgende Schritte:
a) Vorbehandlung der Flüssigkeiten und / oder Suspensionen mittels Ultraschall
b) Behandlung der Flüssigkeiten und / oder Suspensionen mittels einer Kombination aus Ultraschall und Plasma
c) Nachbehandlung der Flüssigkeiten und / oder Suspensionen mittels eines Plasmas.

2. Verfahren nach Anspruch 1, **dadurch gekennzeichnet, dass** die Flüssigkeiten und / oder Suspensionen aus Biogasanlagen oder anderen Fermentationsanlegen entnommen werden und organische und / oder stickstoffhaltige Bestandteile enthalten.

3. Verfahren nach Anspruch 1 oder 2, **dadurch gekennzeichnet, dass** die Flüssigkeiten und / oder Suspensionen einen oder mehrere Prozessschritte 1a) bis 1c) mehrfach durchlaufen können.

4. Verfahren nach einem der Ansprüche 1 bis 3, **dadurch gekennzeichnet, dass** es sich bei den wassersoff-enthaltenden chemischen Verbindungen um Kohlenwasserstoffe, organische Moleküle, Ammoniak, Ammonium-Verbindungen, Schwefelwasserstoff oder Wasserdampf handelt.

5. Verfahren nach einem der Ansprüche 1 bis 4, **dadurch gekennzeichnet, dass** als Plasmen Spark-Plasmen, Corona Plasmen, Microwellen Plasmen oder Torch-Plasmen zur Anwendung kommen und das Plasma partiell Temperaturen von 2000-4000 K erzeugt.

6. Verfahren nach einem der Ansprüche 1 bis 5, **dadurch gekennzeichnet, dass** die behandelten Flüssigkeiten und / oder Suspensionen im Anschluss an das Verfahren der Biogasanlagen oder den anderen Fermentationsanlegen wieder zugeführt werden.

7. Vorrichtung zur Behandlung von Flüssigkeiten und / oder Suspensionen und zur Erzeugung von Wasserstoff aus wassersoff-enthaltenden chemischen Verbindungen, umfassend:
a) mindestens einen Reaktionsraum, in dem Ultraschall-erzeugende Geräte auf die Flüssigkeiten und / oder Suspensionen einwirken und
b) mindestens einen Reaktionsraum, in dem Ultraschall-erzeugende Geräte kombiniert mit plasmaerzeugenden Geräten auf die Flüssigkeiten und / oder Suspensionen einwirken und
c) mindestens einen Reaktionsraum, in dem Plasma-erzeugende Geräte auf die Flüssigkeiten und / oder Suspensionen einwirken
,**dadurch gekennzeichnet, dass** die Flüssigkeiten und / oder Suspensionen nacheinander die Reaktionsräume 7a) bis 7c) durchlaufen.

8. Vorrichtung nach Anspruch 6, **dadurch gekennzeichnet, dass** sie nach den Reaktionsräume 7a) bis 7c) Bypass-Systeme ausweist, um die Flüssigkeiten und / oder Suspensionen nach Passieren eines Reaktionsraums ein weiteres Mal zu behandeln.

9. Verwendung des Verfahrens nach einem der Ansprüche 1 bis 6 oder der Vorrichtung gemäß Anspruch 7 oder 8
a) zur Erzeugung von Wasserstoff oder
b) zur thermischen Dissoziation von Molekülenoder
c) zur Steigerung der Methan-Ausbeute in Biogas-Anlagen oder
d) zum Abbau von Ammoniak, Ammonium-Verbindungen oder Schwefelwasserstoff

10. Verwendung des Verfahrens nach einem der Ansprüche 1 bis 6 oder der Vorrichtung gemäß Anspruch 7 oder 8 im Kombination mit einem Anaerob-Verfahren zur Biomasse Aufbereitung.
